# EUROPEAN PATENT SPECIFICATION

(11) **EP 3 897 825 B1**
(45) Date of publication and mention of the grant of the patent: **09.10.2024**
(21) Application number: 19831933.7
(22) Date of filing: 19.12.2019
(51) Int. Cl.: A61N 1/40

(54) **FLEXIBLE 3D-PRINTED WATER BOLUS INSERTS**
FLEXIBLE 3D-GEDRUCKTE WASSERBOLUSEINSÄTZE
INSERTS DE BOLUS D'EAU FLEXIBLES IMPRIMÉS EN 3D

(30) Priority: 19.12.2018 EP 18213946
(43) Date of publication of application: 27.10.2021
(73) Proprietor: Erasmus University Medical Center Rotterdam, 3015 GE Rotterdam (NL); Technische Universiteit Delft, 2628 CN Delft (NL)
(72) Inventor: PAULIDES, Margarethus Marius, 3015 GE Rotterdam (NL); ABDEL-ALIM-VAN DEN BERG, Anne Lisa, 3015 GE Rotterdam (NL); RUITER, Iemkje A., 2628 CN Delft (NL); BOGERD, Nina, 2628 CN Delft (NL); VAN RHOON, Gerard Cornelis, 3015 GE Rotterdam (NL)
(74) Representative: V.O.
(86) International application number: PCT/NL2019/050858
(87) International publication number: WO 2020/130824

(56) References cited:
- EP-B1- 2 744 564
- WO-A1-2016/210001
- US-B2- 7 582 050
- US-B2- 8 306 628

## Description

### FIELD OF THE INVENTION

The invention relates to a water bolus for covering part of an outer body surface of a body part.

### BACKGROUND

Hyperthermia treatment is a method used for treating tumour cells by exposing body tissue of cancer patients to temperatures in the range of 40-44 °C. Hyperthermia stimulates blood perfusion leading, e.g., to a more effective delivery of drugs in tissues and the presence of more oxygen and, hence, the creation of more oxygen-radicals by ionizing radiation. Hyperthermia also sensitizes tissues to these DNA damaging agents in multiple ways, e.g. through blocking DNA damage repair. Healthy tissue can remain undamaged during hyperthermia treatment up to a temperature of 44 °C for up to an hour. The high temperature that is required for hyperthermia treatment is induced by exposing the patient's body tissue to electromagnetic waves.

While the tumour cells are being heated, the electromagnetic waves may cause skin burns on a patient's skin. Furthermore, electromagnetic waves may be hard to guide to the correct place inside the patient's body. To tackle these issues, a layer with water, a water bolus, is placed between electromagnetic wave generators and the skin of the patient as a cooling agent to prevent burns from the radiated heat and as a transferring agent for the electromagnetic energy. The electromagnetic waves typically have a frequency between 500 kHz and 1 GHz.

Known water boluses are formed as balloons through which water may be circulated. One side of the water bolus is arranged for skin contact, and preferably the contact between water bolus and skin is as good as possible meaning that no air gaps between the water bolus and the skin are desired. The fully water-filled water bolus, however, does not provide a reliable skin contact, as for example folds or sharp wedges occur at the patient side of the water bolus which creates undesired air gaps between the patient's skin and the water bolus. At the site of the wedges the shape change may induce high concentration of the electromagnetic field resulting in local high heating and a toxicity risk to the patient. Next to the high heating, the wedges result in a poorly cooled region which in combination with the heating poses a high risk for burns.

Furthermore, the fully water-filled water bolus presses onto the body part of the patient that is being treated due to the weight and pressure of the water inside the water bolus. This pressing may be experienced as uncomfortable, for example when the body part is the patient's head, face or neck. EP2744564B1 discloses the use of a foam insert, provided inside the water bolus. This foam insert increases the shape stability and decreases the amount of undesired folds at the patient side of the water bolus. Any insert has the disadvantage that it decreases the volume percentage of water in the water bolus which may decrease the volume averaged dielectric value and hence the guidance of the electromagnetic or acoustic energy. Open-cell polyether foam is therefore used since it provides a low filling percentage, and hence allows most of the available volume inside the water bolus to be filled with water. The open cell structure of the foam further allows water to flow easily through the insert and thus through the water bolus which is beneficial for effectively cooling the patient's skin and obtaining a more uniform water temperature within the water bolus.

### Summary

A water bolus is provided with among others an even more improved shape stability.

Although the open-cell foam insert improved shape stability, still unpredictable folds are prone to occur at the patient's skin side of the water bolus. These folds block water circulation locally and inhibit prevention of temperature hotspots on the patient's skin. Furthermore may an increased shape stability of the water bolus provide a better match between a software simulation of the hyperthermia treatment and the real-world situation. Software simulation may be used for planning of the treatment.

A water bolus for covering part of an outer body surface of a body part is provided, comprising a flexible cover forming a container arranged for containing a fluid and an insert provided inside the container, wherein the insert is arranged to provide a first stiffness in a first load direction and a second stiffness in a second load direction, wherein the first stiffness is lower than the second stiffness, and a cover stiffness of the flexible cover is lower than the first stiffness. In an embodiment, the first load direction is perpendicular to the second load direction.

With stiffness, in this description the property is meant of how an object resists deformation under a certain force. The stiffness may be expressed in N/mm, i.e. the amount of force required to deform the object over a certain distance. The stiffness may depend on the material of which an object is made, on the shape of the object, on the wall thickness of the object and in what direction the force is applied on the shape of the object. The insert according to the present invention provides, other than the known open-cell foam inserts, a different stiffness in different load directions. The opposite to stiffness is compliance, i.e. the property of how an object complies to deformation under a certain force.

The open cell foam insert known in the art provides substantially the same stiffness in every load direction, and also substantially the same stiffness no matter where the force is applied because of the structure of an open-cell foam. That structure consists of cells which are not completely encapsulated, which is required for allowing water to flow through the insert.

In the first load direction, more compliance may be required, for example to ensure a good fit between the patient's skin and the water bolus. In the second load direction, more stiffness may be required, for example to resist against shape instability of the water bolus due to the weight and pressure of the water inside the water bolus or to resist against external loads on the water bolus, e.g. handling by the user. The flexible cover which forms the container is generally more compliant than the insert, which means that the shape of the insert pre-dominantly determines the shape of the container and the shape of the flexible cover adapts to the shape of the insert.

In many configurations, the water bolus will have an overhanging part, which is a part which in use is a higher part hanging over a lower part. The higher part will have a tendency to, when the water bolus is filled with water, deform towards the lower part. In use, a patient's head may be present between the lower part and the higher part, and thus the higher part may press into the face of the patient which may be perceived as an uncomfortable pressing. A higher stiffness of the insert against this overhanging may increase the comfort of the patient by reducing the deformation of the higher part towards the lower part and thus relieving pressure on the patient's head. More in general, because the container comprises a flexible cover, the container is prone to sagging when filled with water due to water pressure and weight elastically deforming the flexible cover.

With open cell foam inserts, a single stiffness for all load directions has been chosen due to the isotropic stiffness of the open cell foam material. This leads to either a non-optimal first stiffness in a first load direction, a non-optimal second stiffness in a second load direction, or a compromise between the two stiffnesses resulting in neither an optimal first stiffness nor an optimal second stiffness. A water bolus with an insert with anisotropic stiffness is provided.

At least part of an inner surface of the insert may be substantially complementary to at least part of the outer body surface and the first load direction may be for a substantial part of the inner surface substantially oriented normal to the inner surface of the insert. With the at least part of the inner surface of the insert being complementary to the at least part of the outer body surface, the water bolus is pre-shaped to fit with the outer body surface, which decreases the odds of the undesired folds in the water bolus at the patient's side of the water bolus.

The complementariness of the inner surface of the insert and the outer body surface may result in a small deviation in correspondence between the inner surface of the insert and the outer body surface. The water pressure inflating the container may be used for compensating for the small deviation.

To provide the first stiffness in the first load direction and the second stiffness in a second load direction, the insert may comprise a plurality of linked, thin-walled, elongated, parallel, hollow structures.

The structures are linked for allowing forces to be transferred directly between adjacent structures, and as such for example buckling may be prevented. The structures are thin-walled for minimising the volume of the insert inside the water bolus, which maximises the possible volume of water inside the water bolus. The structures are elongated for allowing an elongated flow-path through the structures, and parallel for having the flowpaths substantially parallel to one another. The structures are hollow to allow a water flow through the structures from one end to the other end.

Within the water bolus, a certain dielectric constant may be preferred. This dielectric constant may be preferred to be relatively high as it may be matched with the electromagnetic frequencies used by a certain hyperthermia device. Additionally or alternatively, the dielectric constant may be matched to a dielectric constant of the body of the patient. To obtain the desired dielectric constant, a certain insert volume to water ratio may be chosen to obtain said desired dielectric constant.

Furthermore, the dielectric constant may differ in different sections of the water bolus, for example by locally adjusting the ratio of insert volume to water volume. The material of which the insert is made typically has a lower dielectric constant than water, and this property may hence be used to at least locally lower the dielectric constant as observed by electromagnetic waves passing through the water bolus towards the patient. Local adjustments to the dielectric constant may for example be made by locally adjusting a wall thickness of the insert. A preferred dielectric constant may be calculated or determined before manufacturing the insert, for example using computer simulation, and as such a specialised insert may be made for a specific case or patient.

When the insert comprises the plurality of linked, thin-walled, elongated, parallel, hollow structures, the second load direction may be oriented substantially parallel to an elongation direction of the hollow structures and the first load direction is substantially perpendicular to the elongation direction of the hollow structures.

In an embodiment of the water bolus, the hollow structures are arranged as elongated hexagonal structures, also known as honeycomb structures. As the name already implies, the honeycomb structure is inspired by the way bees build their beehive to provide enough strength yet as much space as possible for their honey. The hexagonal architecture eliminates unnecessary material which enables a small filling percentage of structure material and a large filling percentage of water inside the water bolus. This ensures a limited influence of material on the electromagnetic waves.

When the inserts are arranged as elongated hexagonal structures, the insert may be arranged to provide a third stiffness in a third load direction, wherein the third load direction is oriented substantially perpendicular to an elongation direction of the hollow structures and at an approximately 90 degree angle relative to the first load direction, and wherein the third stiffness may be higher than the first stiffness. Alternatively, for example by manipulating wall thicknesses of some or all walls of one or more structures, or by manipulating the cross-section of one or more structures, the third stiffness of the insert may be lower than the first stiffness, as will be appreciated by a person skilled in the art. The three load directions may thus be orthogonal relative to each other.

In use of the water bolus, the third load direction may be a substantially vertical direction, and the first load direction may be a substantially horizontal direction.

The container may comprise a fluid input for receiving a fluid and a fluid output for releasing a fluid such that a fluid flow may be provided within the container between the fluid input and the fluid output. Between the fluid input and the fluid output, though the water bolus, a fluid flow may be established. Such a flow allows a better regulation of the fluid temperature in the water bolus, more specifically a more homogenous fluid temperature. The homogenous fluid temperature may be most desired at or near the skin of the patient, and the insert design may be adjusted to optimize a fluid flow arranged for providing this homogenous fluid temperature at or near the skin of the patient.

The preferred fluid is water, but other fluids may also be used in the water bolus. Water has the advantages of being cheap and broadly available, having good thermal properties, and being non-toxic. Depending for example on the desired dielectric constant of the water bolus or sections thereof, different fluids may be chosen.

A first of the fluid input and the fluid output may be provided at a first side of the flexible cover, and a second of the fluid input and the fluid output may be provided by a fluid conduit substantially extending from the first side to a second side of the flexible cover opposite to the first side.

With this configuration of the fluid input and the fluid output, both may be provided on the same side of the water bolus whilst maintaining a good water flow through the entire water bolus.

When the second of the fluid input and the fluid output is provided by the fluid conduit, the fluid conduit may extend through the container and thus not outside the container. This may provide a compact form-factor of the water bolus, and both at the patient side and the device side there is no room for extending the fluid conduit outside the water bolus.

At least some of the plurality of hollow structures may comprise a plurality of perforations such that hollow interiors of adjacent hollow structures provided with the perforations are in fluid communication with one another. This allows not only a separate fluid flow through each individual elongated structure, but also a fluid flow between adjacent structures. This may increase the homogeneity of the water temperature inside the water bolus.

The water bolus fluid may be arranged to have fluid flowing within the water bolus from the first side to the second side and a flow-through surface of the perforations may substantially increase from the first side to the second side.

To be able to measure the pressure applied on the patient's skin, a pressure sensor can be inserted inside the water bolus. Based on the measured pressure, the water volume inside the bolus can be increased or decreased.

The water bolus may be arranged for transferring electromagnetic energy through the water bolus, for example when the water bolus is to be used in hyperthermia treatment.

A wall thickness of the hollow structures may increase dependent on expected fluid pressures inside the water bolus. This allows a further optimization of the volume occupied by the insert inside the water bolus. Not everywhere is the same wall thickness required, since the forces due to water pressure will increase in a direction opposite to gravity.

When the water bolus is arranged for a fluid flow within the water bolus from the first side to the second side, perforations may be spaced apart, and an average distance between adjacent perforations may increase from the first side to the second side.

When the structures comprise multiple perforations, a first perforation may be provided out of register with a second perforation relative to the direction of elongation. This may provide a flow path through the perforations with enhanced thermal properties for the water bolus.

### BRIEF DESCRIPTION OF THE FIGURES

The various aspects and embodiments thereof will now be discussed in conjunction with drawings. In the drawings:
FIG. 1A shows a top view of part of a hyperthermia device;
FIG. 1B shows a front view of part of a hyperthermia device;
FIG. 2A shows another top view of part of a hyperthermia device;
FIG. 2B shows another front view of part of a hyperthermia device;
FIG. 3A shows part of an embodiment of an insert;
FIG. 3B shows another embodiment of an insert;
FIG. 4A shows a schematic view of part of a hyperthermia device; and
FIG. 4B shows another schematic view of part of a hyperthermia device.

### DETAILED DESCRIPTION OF THE EMBODIMENTS

Different embodiments of the present invention will be explained in more detail based on an example of a use of the water bolus in hyperthermia treatment. Different applications for the water bolus are envisioned as well, as will be discussed further onwards in this description.

One of the most difficult areas to reach with hyperthermia treatment is the head and neck region, and the figures will therefore be directed towards embodiments of a water bolus for use in hyperthermia treatment for head, neck, and/or larynx. It will be appreciated by a person skilled in the art that different applications of the water bolus for different body parts are also envisioned, and thus that the water bolus may be shaped differently than shown in the figures. For example, a water bolus for use in treatment in the head region may span a distance between the eyes and the chin of a patient. In another example, a water bolus for use in treatment in the neck region may span a distance between the eyes and the collarbone of the patient. The height of the water bolus may for example reach from the back of the head of the patient up to the height of the eye level of the patient. In yet another example, the water bolus may be shaped complementary to one or more other body parts, such as a skull, a belly, at least part of a leg, at least part of a pelvic area, breasts, any other body part, in particular curved body part, or any combination thereof. A shaped water bolus may be pre-shaped such that a desired shape is achieved after filling the water bolus with water. Computer calculations and/or simulations may be used for determining the shape of the water bolus in a situation without water, and in a situation wherein the water bolus has been filled with water. The position and/or orientation in which the water bolus will be used may be taken into account as well, as gravity may affect the shape of the water bolus.

The main aims of the water bolus are as discussed the transferring of electromagnetic waves to the patient's tissue, and preventing skin burns on the patient's skin due to heating caused by these electromagnetic waves. It is further an aim of the water bolus to assist in maintaining a position and/or orientation of the head of the patient during the treatment.

Next to being more comfortable for patients, the water bolus according to the present invention may be more easily useable by an operator of a hyperthermia device who has to handle the water bolus because of the increased stiffness in certain load directions. The more easy useability may result in one or more of: a higher reproducibility of the hyperthermia treatment between different treatment settings, a better transfer of predicted energy patterns to the patient, and a better treatment outcome. Furthermore may a faster set-up of the hyperthermia device be achieved, which may save time and enhance acceptance of the treatment by the patient.

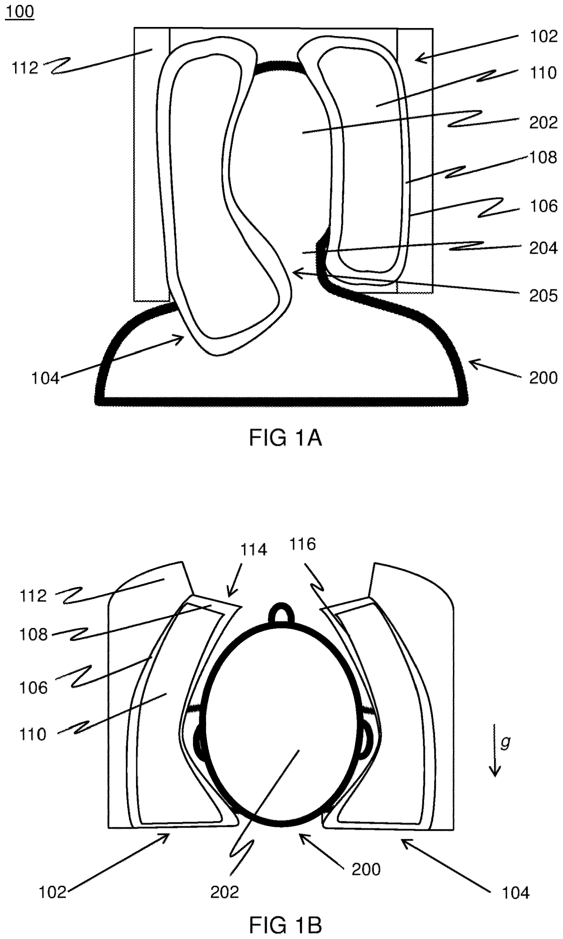

Fig. 1A shows a top view of part of a hyperthermia device 100 with part of a patient 200 lying inside the device 100. The hyperthermia device 100 comprises two separate water boluses: a left water bolus 102 and a right water bolus 104, with left and right as seen by the patient. The left water bolus 102 is arranged for covering part of an outer surface of a head 202, a neck 204 and a larynx 205 as outer body surfaces of body parts. The right water bolus 104 is arranged for covering an outer surface of the head 202 and the neck 204 as outer body surfaces of body parts. For conciseness of the description and because the two water boluses function similarly, from here on only the left water bolus 102 will be discussed and will be referred to as water bolus 102. The water boluses may be supported by a frame 112 comprised by the hyperthermia treatment device 100.

Not shown in the figures are radiation sources comprised by the hyperthermia device 100, wherein the radiation sources are arranged to direct electromagnetic radiation towards the patient through the water bolus 102. In use, the water bolus 102 will be provided between the patient's body part, which will receive electromagnetic radiation and the radiation sources.

The water bolus 102 comprises a flexible cover 106 forming a container 108 arranged for containing a fluid such as water, and an insert 110 provided inside the container, wherein the insert 110 is arranged to provide a first stiffness in a first load direction and a second stiffness in a second load direction, wherein the first stiffness is lower than the second stiffness, and a stiffness of the flexible cover is lower than the first stiffness.

Fig. 1B shows a front view of the hyperthermia device 100 and the back of the head 202 of the patient 200. The left water bolus 102 and the right water bolus 104 surround the head 202 and have for clarity of the figure been slightly moved outward away from the head 202. In use, the water bolus 102 will be placed in contact with the patient's skin.

Fig. 1B shows an overhanging part 114 of the water bolus 102, which due to the weight of the water inside the water bolus 102 will want to collapse in a direction opposite to the gravity g. In Fig. 1B, the inner shape 116 of the insert 110 is also visible, which corresponds to an outer body surface.

As can been seen from Figs. 1A and 1B, it is preferred to leave at least some part of the patient's face exposed. This allows the patient to breathe through his nose and/or mouth, and preferably allow the patient's eyes to remain exposed as well. The exposure increases comfort of the patient, and decreases the feeling of being trapped inside a device for the patient.

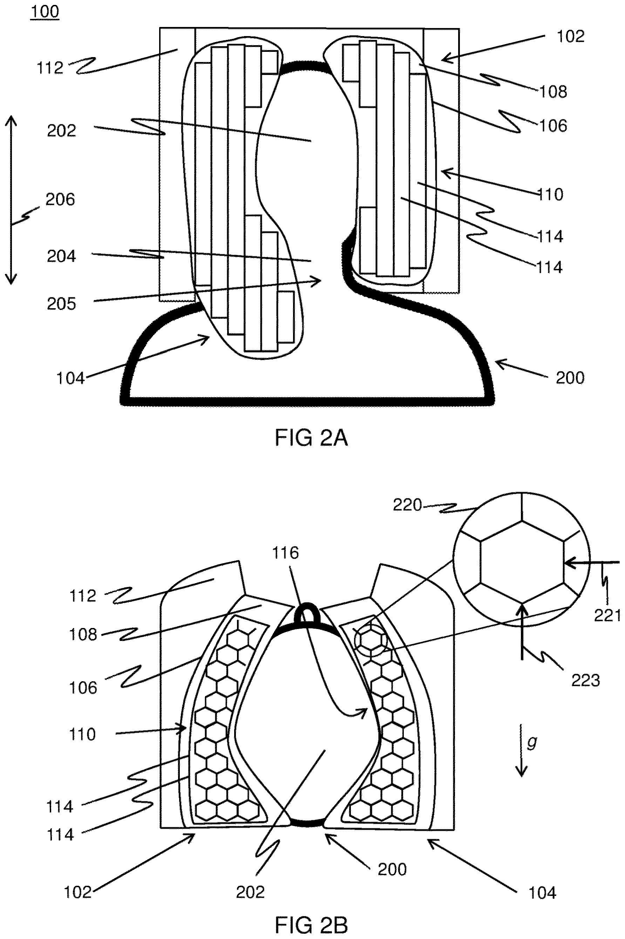

Fig. 2A again shows a top view of part of a hyperthermia device 100 with part of the patient 200 lying inside the device 100. In the embodiment of the water bolus 102 as shown in Fig. 2A, the insert 110 comprises a plurality of linked, thin-walled, elongated, parallel, hollow structures 114. In the embodiment of Fig. 2A, an elongation direction 206 of the structures 114 is substantially parallel to the length of the patient 200 from head to toe. Embodiments of the insert wherein the elongation direction 206 is perpendicular to the length of the patient are envisioned as well.

A cross-section of an elongated structure may be constant over the length of the structure, and may have any shape such as a triangle, rectangle, square, circle, hexagonal, any other shape, or any combination thereof. Embodiments of the insert 110 comprising elongated structures with different cross-sections are envisioned as well, and the cross-section of one structure need not be constant over the length of the structure.

Fig. 2B shows a view of the back of the head 202 of the patient 200, wherein the head 202 is partially surrounded by the water boluses 102 and 104. In Fig. 2B, the elongation direction 206 extends into the plane of the paper. The structures 114 of the insert 110 are in this embodiment hexagonally shaped. The structures 114 are in the embodiment of Fig. 2B provided such that two straight side planes of the hexagonal structures are substantially parallel to the direction of the gravity g. Embodiments of the insert 110 wherein the structures 114 are rotated 90 degrees or any other angle over the elongation direction are also envisioned.

Hexagonal structures 114 provide a first stiffness in a first load direction, a second stiffness in a second load direction, and a third stiffness in a third load direction. The first load direction, indicated with 221 in detail 220 of Fig. 2B, may be a direction perpendicular to one of the planes comprised by the hexagonal, and may in use be substantially perpendicular to the direction of gravity g. A second load direction may be parallel to the elongation direction 206 of the hexagonal structure. The third load direction, indicated with 223 in detail 220, may be a direction angled 90 degrees relative to the first load direction 221 around an axis parallel to the elongation direction 206. This corresponds to a load on the line where two planes of the hexagonal meet.

Depending on the material choice for the structures and the wall thicknesses of the structures, the first stiffness may be lower than the second stiffness, and the third stiffness may be higher than the first stiffness. Using this knowledge, a person skilled in the art may position the structures in the water bolus 102 as preferred. For adjusting the stiffness of the insert 110 in any direction, a person skilled in the art will appreciate different ways of manipulating properties of the structures such as wall thicknesses, orientation, and cross-sectional shapes to achieve a desired stiffness in a certain direction and/or a ratio between different stiffnesses in different directions.

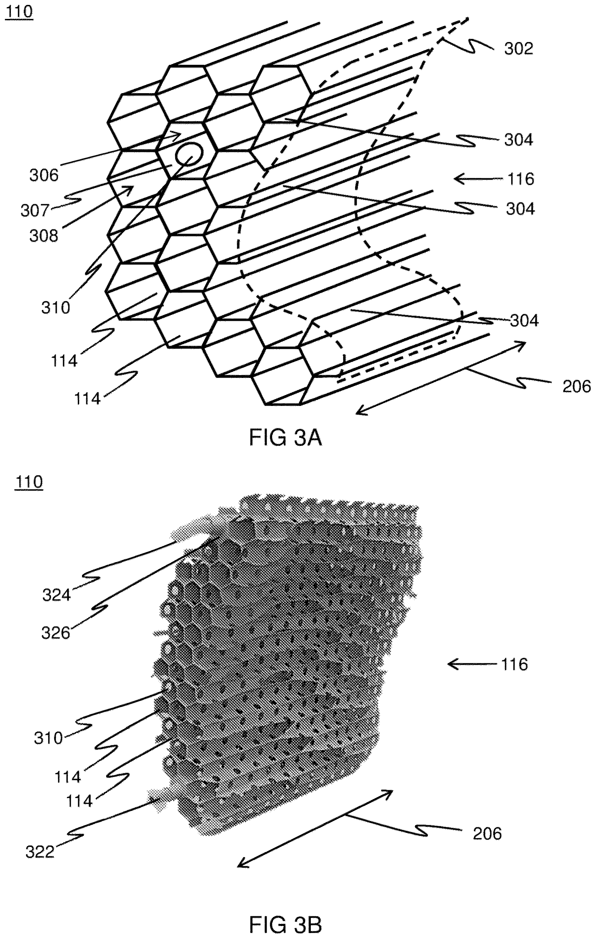

Fig. 3A shows part of an embodiment of an insert 110 for a water bolus, which insert 110 comprises a plurality of linked, thin-walled, elongated, parallel, hollow, hexagonal structures 114. The inner shape 116 substantially corresponds to an outer body surface indicated by the dotted contour 302. Because the dotted contour 302 may not perfectly correspond to an insert 110 shape made out of only full hexagonal structures, some of the structures are only partially provided as partial structures 304 with only one, two, three. four or five out of the six possible planes present.

Instead of, or next to, using the partial structures 304, structures 114 with different cross-sectional shapes and/or dimensions may be comprised by the insert 110. For example, to follow the shape of the dotted contour 302 better, the insert 110 may comprise at the side of the inner shape 116 structures with a smaller cross-sectional surface. This allows the insert to comprise less partial structures 304, and thus more full structures. Furthermore, for example to follow the shape of the dotted contour 302 better, one or more structures 114 may comprise a non-constant cross-sectional shape.

Next to a non-constant cross-sectional shape, or as an option on itself, one or more structures 114 comprised by insert 110 may have a non-constant wall thickness. A locally higher wall thickness may increase the shape stability of the entire insert 110. Keeping this in mind, a person skilled in the art may readily appreciate how to adapt the wall thickness and/or cross-sectional shape of one or more structures 114 of the insert 110.

A first structure 306 and a second structure 308 as adjacent structures share a common plane 307 which comprises a perforation 310. By virtue of perforation 310, the first structure 306 and the second structure 308 are in fluid communication and fluid may flow between the first structure 306 and the second structure 308 through the perforation 310. Any structure may comprise any number of perforations in any plane of the structure, for example any of the six planes when the structures are shaped as hexagonal structures. Only one perforation 310 is shown in Fig. 3A for clarity an conciseness of the figure.

An embodiment of the insert 110 similar to that of the insert 110 shown in Fig. 3A is also envisioned wherein the hexagonal structures 114 are rotated 90 degrees around the elongation direction 206, i.e. with the flat sides of the hexagonal shape not pointing up and down but left and right. Such an embodiment is shown in Fig. 3B, which shows a rendering of an embodiment of the insert 110.

The insert of Fig. 3B comprises a plurality of linked, thin-walled, elongated, parallel, hollow, and hexagonal structures 114. The insert 110 further comprises a plurality of perforations 310 provided in the structures 114. As can be seen in Fig. 3B, a large number of perforations 310 may be provided on any and all planes of the hexagonal structures 114.

The insert 110 may further comprise a fluid inlet 322 and a fluid outlet 324. The fluid inlet 322 may be used when filling the water bolus 102 with a fluid such as water, and the fluid inlet 322 may be used for draining the water bolus 102. Both the fluid inlet 322 and the fluid outlet 324 may be used for water circulation through the water bolus.

When the fluid inlet 322 is in use provided lower than the fluid outlet 324 relative to the gravity, filling the water bolus with fluid using the fluid inlet 322 allows air to escape the water bolus through the fluid outlet 324. While draining the water bolus, gravity will assist the fluid to leave the water bolus through the lower fluid inlet 322.

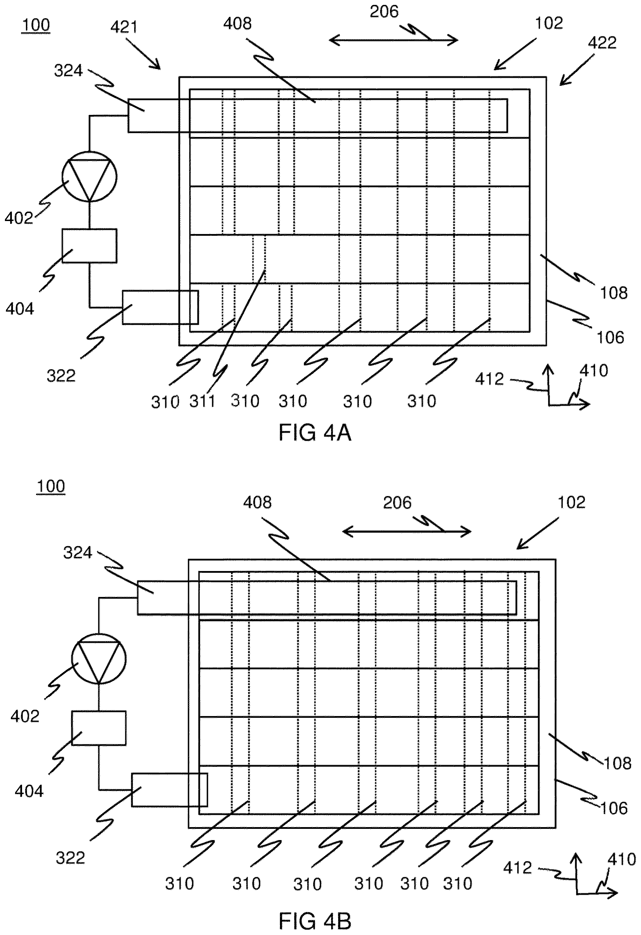

Fig. 4A shows a schematic view of part of a hyperthermia device 100 to indicate how water as an example of a fluid may flow through embodiments of the water bolus 102. The hyperthermia device 100 further comprises a pumping device 402 for pumping the liquid through the water bolus 102 as a flow of water and an optional temperature regulator 404 for controlling a temperature of the water flowing through towards the water bolus 102. The temperature regulator 404 may be provided downstream or upstream of the pumping device 402.

For receiving the flow of water the water bolus 102 comprises a fluid input 322, and for creating the flow path towards the pumping device 402. The water bolus 102 further comprises the fluid output 324. In the embodiment of Fig. 4A, the fluid input 322 and the fluid output 324 are provided at the same side of the flexible cover 106 of the water bolus 102, which may be a first side.

It is preferred that the fluid flow comprises two directional components: a first flow direction 410 substantially corresponding to the elongation direction 206 of the structures 114, and a second flow direction 412, perpendicular to the first flow direction 410. Flow in the first flow direction 410 is allowed through the hollow structures 114, and flow in the second flow direction 412 is allowed through the plurality of perforations 310, indicated with dotted lines.

In the embodiment of Fig. 4A, a flow-through surface of the perforations 310 substantially increases from the first side 421 to the second side 422. With this increasing, fluid flow in the second flow direction 412 becomes more easy the further the flow is away from the first side 421 where the pumping device 402 is provided. A more uniform fluid flow inside the water bolus 102 may be achieved, and as a result thereof a more uniform temperature distribution inside the water bolus 102 may be achieved as well.

Perforations 310 of a structure may be aligned with perforations 310 of a structure adjacent to it in the second flow direction 412. Alternatively, or additionally, some perforations 310 may be shifted, such as the exemplary shifted perforation 311, which is shifted along the first flow direction 410 relative to the other perforations 310. As can been seen in Fig. 4A, this shifted perforation 311 is not aligned with the perforations 310 of adjacent structures in the second direction 412. Such a misalignment may prevent a direct fluid flow through vertically adjacent perforations 310 and increase homogeneity of fluid flow and temperature inside the water bolus 102.

The water bolus 102 may be provided with a fluid conduit 408 substantially extending from the first side 421 to the second side 422 of the flexible cover 106. The fluid conduit 408 may shift the fluid output 324 from the first side 421 towards the second side 422. With this shifting, it becomes easier for the fluid flow to reach the second side 422 of the water bolus 102 with sufficient flow capacity. Although the fluid conduit 408 may be provided partially outside the container 108, it is preferred that the fluid conduit 408 extends through the container 108 for compactness of the water bolus 102.

The embodiment of the part of the hyperthermia device 100 as shown in Fig. 4B substantially corresponds to the embodiment of the part of the hyperthermia device 100 as shown in Fig. 4A. However, in the embodiment of Fig. 4B, an average distance between adjacent perforations 310 decreases from the first side 421 to the second side 422. With this decreasing average distance between adjacent perforation 310 more flow through area is provided for the fluid flow in the second flow direction 412 the further away from the first side 421 the fluid flow is. This may also provide a more uniform fluid flow inside the water bolus 102, and as a result thereof a more uniform temperature distribution inside the water bolus 102.

An embodiment of the water bolus 102 is also envisioned wherein both the flow through area of the perforations 310 increases and the average distance between adjacent perforations 310 decreases from the first side 421 to the second side 422. This combination may even further provide a more uniform fluid flow inside the water bolus 102, and as a result thereof an even more uniform temperature distribution inside the water bolus 102.

In use, the first side 421 may correspond to the side to which the top of the head of the patient is pointing. The second side 422, opposite to the first side, may then correspond to the side of the feet of the patient. In such an embodiment, all fluid conduits may be provided out of sight and out of reach of the patient.

In embodiments of the water bolus 102, the fluid input 322 and fluid output 324 as shown in Figs. 4A and 4B may be interchanged, and the flow direction may then be opposite to that as described here above.

Embodiments of the insert 110 are also envisioned wherein the structures 114 are rotated 90 degrees relative to the structures 114 shown in Figs. 4A and 4B. As a result thereof, the fluid flow in the first flow direction 410 is allowed through the perforations 310, and the fluid flow in the second flow direction 412 is allowed though the hollow structures 114.

Any of the embodiments of the insert 110 may be manufactured using 3D-printing. This manufacturing technique allows for an insert 110 design which corresponds at one side to the hyperthermia device and another side to the outer surface of a body part. A 3D model may first be made of the body part, or an average model may be constructed from multiple 3D models. Using such a body model, the inner shape 116 of the insert 110 may be determined either for a specific patient, or for an average set of patients. When using 3D-printing, CAD models may be used for both the patient models and the insert 110 models.

It is also envisioned to print the insert 110 and the cover 106 simultaneously, optionally as a single part. Different 3D printing techniques known to a person skilled in the art may be used for this purpose, such as using support material which may be soluble, and printing in spiral, spiralizer, or spiral vase mode for printing the cover 106.

Although the embodiments of this description have been directed towards the use of the water bolus in hyperthermia treatment in the head and neck region, other applications even outside hyperthermia treatment are envisioned as well for the water bolus according to the present invention.

Examples of such applications are: temperature regulation, i.e. heating and/or cooling, of outer surfaces of body parts of a human or animal. For example, heating or cooling of the neck using a water bolus according to the present invention may alter the thermoregulation in patients by influencing the temperature in the thalamus. Skin cooling may also reduce the impact of drugs in the skin, and neck heating may stabilise the temperature of a patient during anaesthesia. Also in sports or physiotherapy may the water bolus with increased shape stability be used.

In medical applications other than hyperthermia treatment a water bolus according to the present invention may be placed between medical equipment and a patient.

In summary, during hyperthermia treatment of the head and neck area, a conventional water bolus, used in such treatment for guiding electromagnetic waves and cooling the patient's skin, may collapse onto the patient's face due to the weight and/or pressure of the water inside the water bolus. An insert may be provided inside the water bolus to remedy this, wherein the insert provides additional stiffness and thus resistance against the water's weight and pressure. The insert however should not be too resistant against the shape of the patient, and thus an insert is provided with a first stiffness in a first load direction and a second stiffness in a second load direction, wherein the first stiffness is not equal to the second stiffness.

In the description above, it will be understood that when an element such as layer, region or substrate is referred to as being "on" or "onto" another element, the element is either directly on the other element, or intervening elements may also be present. Also, it will be understood that the values given in the description above, are given by way of example and that other values may be possible and/or may be strived for.

Furthermore, the invention may also be embodied with less components than provided in the embodiments described here, wherein one component carries out multiple functions. Just as well may the invention be embodied using more elements than depicted in the Figures, wherein functions carried out by one component in the embodiment provided are distributed over multiple components.

It is to be noted that the figures are only schematic representations of embodiments of the invention that are given by way of non-limiting examples. For the purpose of clarity and a concise description, features are described herein as part of the same or separate embodiments, however, it will be appreciated that the scope of the invention may include embodiments having combinations of all or some of the features described. The word 'comprising' does not exclude the presence of other features or steps than those listed in a claim. Furthermore, the words 'a' and 'an' shall not be construed as limited to 'only one', but instead are used to mean 'at least one', and do not exclude a plurality.

A person skilled in the art will readily appreciate that various parameters and values thereof disclosed in the description may be modified and that various embodiments disclosed and/or claimed may be combined without departing from the scope of the invention.

It is stipulated that the reference signs in the claims do not limit the scope of the claims, but are merely inserted to enhance the legibility of the claims.

## Claims

1. Water bolus (102, 104) for covering part of an outer body surface of a body part, comprising:
- a flexible cover (106) forming a container arranged for containing a fluid; and
- an insert (110) provided inside the container, wherein the insert is arranged to provide a first stiffness in a first load direction and a second stiffness in a second load direction, **characterized in that** the first stiffness is lower than the second stiffness, and a stiffness of the flexible cover is lower than the first stiffness.

2. Water bolus according to claim 1, wherein an inner surface of the insert is complementary to the outer body surface and the first load direction is for a substantial part of the inner surface substantially oriented normal to the inner surface of the insert.

3. Water bolus according to claim 1 or 2, wherein the insert comprises a plurality of linked, thin-walled, elongated, parallel, hollow structures.

4. Water bolus according to claim 3, wherein the second load direction is oriented substantially parallel to an elongation direction of the hollow structures and the first load direction is substantially perpendicular to the elongation direction of the hollow structures.

5. Water bolus according to claims 2-4, wherein the hollow structures are arranged as elongated hexagonal structures.

6. Water bolus according to claim 5, wherein the insert is arranged to provide a third stiffness in a third load direction, wherein the third load direction is oriented substantially perpendicular to an elongation direction of the hollow structures and at an approximately 90 degree angle relative to the first load direction, and wherein the third stiffness is higher than the first stiffness.

7. Water bolus according to claim 6, wherein in use, the third load direction is a substantially vertical direction, and the first load direction is a substantially horizontal direction.

8. Water bolus according to any of the preceding claims, wherein the container comprises a fluid input for receiving a fluid and a fluid output for releasing a fluid such that a fluid flow may be provided within the container between the fluid input and the fluid output.

9. Water bolus according to claim 8, wherein a first of the fluid input and the fluid output is provided at a first side of the flexible cover, and a second of the fluid input and the fluid output is provided by a fluid conduit substantially extending from the first side to a second side of the flexible cover opposite to the first side.

10. Water bolus according to claim 9, wherein the fluid conduit extends through the container.

11. Water bolus according to any of the preceding claims, wherein the hollow structures comprise a plurality of perforations such that hollow interiors of adjacent hollow structures are in fluid communication with one another.

12. Water bolus according to claim 11 to the extend dependent on claims 8-10, wherein:
- fluid is arranged to flow within the water bolus from the first side to the second side; and
- a flow-through surface of the perforations substantially increases from the first side to the second side.

13. Water bolus according to claims 11 or 12 to the extend dependent on claims 8-10, wherein a first perforation is provided out of register with a second perforation relative to the direction of elongation.

14. Water bolus according to any of the claims 8-13, wherein fluid is arranged to flow within the water bolus from the first side to the second side, the perforations are spaced apart, and an average distance between adjacent perforations increases from the first side to the second side.

15. Water bolus according to claims 3-14, wherein a wall thickness of the hollow structures increases dependent on expected fluid pressures inside the water bolus.

## Patentansprüche

1. Wasserbolus (102, 104) zum Abdecken eines Teils einer äußeren Körperoberfläche eines Körperteils, umfassend:
- eine flexible Abdeckung (106), die einen Behälter bildet, der zum Enthalten eines Fluids angeordnet ist; und
- einen Einsatz (110), der im Inneren des Behälters bereitgestellt ist, wobei der Einsatz angeordnet ist, um eine erste Steifigkeit in einer ersten Belastungsrichtung und eine zweite Steifigkeit in einer zweiten Belastungsrichtung bereitzustellen, **dadurch gekennzeichnet, dass** die erste Steifigkeit niedriger als die zweite Steifigkeit ist, und eine Steifigkeit der flexiblen Abdeckung niedriger als die erste Steifigkeit ist.

2. Wasserbolus nach Anspruch 1, wobei eine innere Oberfläche des Einsatzes komplementär zu der äußeren Körperoberfläche ist und die erste Belastungsrichtung für einen wesentlichen Teil der inneren Oberfläche im Wesentlichen normal zu der inneren Oberfläche des Einsatzes ausgerichtet ist.

3. Wasserbolus nach Anspruch 1 oder 2, wobei der Einsatz eine Vielzahl von miteinander verbundenen, dünnwandigen, länglichen, parallelen, hohlen Strukturen umfasst.

4. Wasserbolus nach Anspruch 3, wobei die zweite Belastungsrichtung im Wesentlichen parallel zu einer Dehnungsrichtung der hohlen Strukturen ausgerichtet ist, und die erste Belastungsrichtung im Wesentlichen senkrecht zu der Dehnungsrichtung der hohlen Strukturen verläuft.

5. Wasserbolus nach einem der Ansprüche 2 bis 4, wobei die hohlen Strukturen als langgestreckte hexagonale Strukturen angeordnet sind.

6. Wasserbolus nach Anspruch 5, wobei der Einsatz angeordnet ist, um eine dritte Steifigkeit in einer dritten Belastungsrichtung bereitzustellen, wobei die dritte Belastungsrichtung im Wesentlichen senkrecht zu einer Dehnungsrichtung der hohlen Strukturen und in einem Winkel von ungefähr 90 Grad relativ zu der ersten Belastungsrichtung ausgerichtet ist und wobei die dritte Steifigkeit höher ist als die erste Steifigkeit.

7. Wasserbolus nach Anspruch 6, wobei bei Verwendung die dritte Belastungsrichtung eine im Wesentlichen vertikale Richtung ist und die erste Belastungsrichtung eine im Wesentlichen horizontale Richtung ist.

8. Wasserbolus nach einem der vorstehenden Ansprüche, wobei der Behälter einen Fluideingang zum Aufnehmen eines Fluids und einen Fluidausgang zum Abgeben eines Fluids umfasst, sodass ein Fluidstrom innerhalb des Behälters zwischen dem Fluideingang und dem Fluidausgang bereitgestellt werden kann.

9. Wasserbolus nach Anspruch 8, wobei ein erster des Fluideingangs und des Fluidausgangs an einer ersten Seite der flexiblen Abdeckung bereitgestellt ist, und ein zweiter des Fluideingangs und des Fluidausgangs durch eine Fluidleitung bereitgestellt ist, die sich im Wesentlichen von der ersten Seite zu einer zweiten Seite der flexiblen Abdeckung gegenüber der ersten Seite erstreckt.

10. Wasserbolus nach Anspruch 9, wobei sich die Fluidleitung durch den Behälter erstreckt.

11. Wasserbolus nach einem der vorstehenden Ansprüche, wobei die hohlen Strukturen eine Vielzahl von Perforationen umfassen, sodass hohle Innenräume benachbarter hohler Strukturen in Fluidverbindung miteinander stehen.

12. Wasserbolus nach Anspruch 11 in dem von den Ansprüchen 8 bis 10 abhängigen Umfang, wobei:
Fluid angeordnet ist, um innerhalb des Wasserbolus von der ersten Seite zur zweiten Seite zu fließen; und
eine Durchflussoberfläche der Perforationen im Wesentlichen von der ersten Seite zur zweiten Seite zunimmt.

13. Wasserbolus nach Anspruch 11 oder 12 in dem von den Ansprüchen 8 bis 10 abhängigen Umfang, wobei eine erste Perforation außer Deckung mit einer zweiten Perforation in Bezug auf die Dehnungsrichtung bereitgestellt ist.

14. Wasserbolus nach einem der Ansprüche 8 bis 13, wobei das Fluid angeordnet ist, um innerhalb des Wasserbolus von der ersten Seite zu der zweiten Seite zu fließen, die Perforationen voneinander beabstandet sind, und ein durchschnittlicher Abstand zwischen benachbarten Perforationen von der ersten Seite zu der zweiten Seite zunimmt.

15. Wasserbolus nach einem der Ansprüche 3 bis 14, wobei eine Wanddicke der hohlen Strukturen in Abhängigkeit von den erwarteten Fluiddrücken im Inneren des Wasserbolus zunimmt.

## Revendications

1. Bolus d'eau (102, 104) pour recouvrir une partie d'une surface de corps externe d'une partie corporelle, comprenant :
une couverture flexible (106) formant un récipient agencé pour contenir un fluide ; et
un insert (110) prévu à l'intérieur du récipient, dans lequel l'insert est agencé pour fournir une première rigidité dans une première direction de charge et une deuxième rigidité dans une deuxième direction de charge, **caractérisé en ce que** :
la première rigidité est inférieure à la deuxième rigidité, et une rigidité de la couverture flexible est inférieure à la première rigidité.

2. Bolus d'eau selon la revendication 1, dans lequel une surface interne de l'insert est complémentaire de la surface de corps externe et la première direction de charge est pour une partie sensible de la surface interne sensiblement orientée de manière normale à la surface interne de l'insert.

3. Bolus d'eau selon la revendication 1 ou 2, dans lequel l'insert comprend une pluralité de structures reliées, à fine paroi, allongées, parallèles, creuses.

4. Bolus d'eau selon la revendication 3, dans lequel la deuxième direction de charge est orientée de manière sensiblement parallèle à une direction allongée des structures creuses et la première direction de charge est sensiblement perpendiculaire à la direction d'allongement des structures creuses.

5. Bolus d'eau selon les revendications 2 à 4, dans lequel les structures creuses sont agencées comme des structures hexagonales allongées.

6. Bolus d'eau selon la revendication 5, dans lequel l'insert est agencé pour fournir une troisième rigidité dans une troisième direction de charge, dans lequel la troisième direction de charge est orientée de manière sensiblement perpendiculaire à une direction d'allongement des structures creuses et à un angle d'approximativement 90 degrés par rapport à la première direction de charge, et dans lequel la troisième rigidité est supérieure à la première rigidité.

7. Bolus d'eau selon la revendication 6, dans lequel, à l'usage, la troisième direction de charge est une direction sensiblement verticale, et la première direction de charge est une direction sensiblement horizontale.

8. Bolus d'eau selon l'une quelconque des revendications précédentes, dans lequel le récipient comprend une entrée de fluide pour recevoir un fluide et une sortie de fluide pour libérer un fluide de sorte qu'un flux de fluide peut être prévu à l'intérieur du récipient entre l'entrée de fluide et la sortie de fluide.

9. Bolus d'eau selon la revendication 8, dans lequel une première parmi l'entrée de fluide et la sortie de fluide est prévue au niveau d'un premier côté de la couverture flexible, et une deuxième parmi l'entrée de fluide et la sortie de fluide est fournie par un conduit de fluide s'étendant sensiblement du premier côté à un deuxième côté de la couverture flexible opposé au premier côté.

10. Bolus d'eau selon la revendication 9, dans lequel le conduit de fluide s'étend à travers le récipient.

11. Bolus d'eau selon l'une quelconque des revendications précédentes, dans lequel les structures creuses comprennent une pluralité de perforations de sorte que les intérieurs creux des structures creuses adjacentes sont en communication de fluide les uns avec les autres.

12. Bolus d'eau selon la revendication 11 dans la mesure où elle dépend des revendications 8 à 10, dans lequel :
le fluide est agencé pour s'écouler à l'intérieur du bolus d'eau du premier côté au deuxième côté ; et
une surface traversante des perforations augmente sensiblement du premier côté au deuxième côté.

13. Bolus d'eau selon la revendication 11 ou 12 dans la mesure où elle dépend des revendications 8 à 10, dans lequel une première perforation est prévue de manière décalée avec une deuxième perforation par rapport à la direction d'allongement.

14. Bolus d'eau selon l'une quelconque des revendications 8 à 13, dans lequel du fluide est agencé pour s'écouler à l'intérieur du bolus d'eau du premier côté au deuxième côté, les perforations sont espacées et une distance moyenne entre les perforations adjacentes augmente du premier côté au deuxième côté.

15. Bolus d'eau selon les revendications 3 à 14, dans lequel une épaisseur de paroi des structures creuses augmente en fonction des pressions de fluide attendues à l'intérieur du bolus d'eau.
